# FASCICULE DE BREVET EUROPEEN

(11) **EP 0 664 715 B1**
(45) Date de publication et mention de la délivrance du brevet: **12.06.1996**
(21) Numéro de dépôt: 93920904.5
(22) Date de dépôt: 21.09.1993
(51) Int. Cl.: A61L 11/00, A61L 2/20

(54) **DISPOSITIF DE TRAITEMENT NOTAMMENT DE DECONTAMINATION DE MATERIAUX DE PREFERENCE SOLIDES TELS QUE DES DECHETS**
BEHANDLUNGSEINRICHTUNG INSBESONDERE FÜR DIE DEKONTAMINIERUNG VON FESTSTOFFEN WIE MÜLL
TREATMENT DEVICE PARTICULARLY FOR DECONTAMINATING MATERIALS, PREFERABLY SOLIDS SUCH AS WASTE MATERIALS

(30) Priorité: 22.09.1992 FR 9211259
(43) Date de publication de la demande: 02.08.1995
(73) Titulaire: Carba Société Anonyme, CH-3097 Liebefeld (CH)
(72) Inventeur: DUROSELLE, Patrick, F-44800 Saint-Herblain (FR); HELD, Bernard, F-64000 Pau (FR); PEYROUS, René, F-64121 Serres-Castet (FR); MONGE, Catherine, F-64000 Pau (FR); PETILLOT, Serge, F-44230 Saint-Sébastien-Sur-Loire (FR)
(74) Mandataire: BOVARD AG - Patentanwälte
(86) Numéro de dépôt international: FR9300908
(87) Numéro de publication internationale: WO9406483

(56) Documents cités:
- EP-A- 0 281 870
- US-A- 4 156 652
- US-A- 5 087 419

## Description

La présente invention concerne un dispositif de traitement, en particulier de décontamination de matériaux, de préférence solides, tels que des déchets provenant par exemple du milieu médical ainsi qu'un procédé de mise en oeuvre de ce dispositif.

Les praticiens, en particulier libéraux, des professions médicales et paramédicales, engendrent un certain nombre de détritus de nature physique diverse (plastique, textile, metal, verre, etc.) tous susceptibles d'être contaminés par les germes microbiens ou viraux provenant de leurs patients.

La quantité de ces déchets est en constante augmentation avec, d'une part le développement des consommations de la santé, et d'autre part, avec l'utilisation par les hommes de l'art d'une quantité sans cesse croissante d'objets à usage unique qui n'ont ainsi plus besoin d'être stérilisés après leur utilisation et leur contamination.

Pour pallier ces inconvénients, un certain nombre de dispositifs d'incinération ont été mis au point. Ils nécessitent cependant l'organisation d'une collecte des déchets et la disposition à proximité d'un dispositif de ce type.

Outre des procédés et dispositifs faisant appel à la chaleur, on a vu se développer ces dernières années des dispositifs mettant en oeuvre soit des solutions chimiques ou physiques. Les dispositifs basés sur l'action d'agents chimiques offrent l'intérêt de permettre des installations plus légères. Ainsi, il est connu un appareil qui, dans une première étape broie les déchets, les réduisant à une granulométrie standard. Ensuite, ce granulat est plongé pour un temps déterminé dans une solution désinfectante puis, après égouttage, il est compacté au moyen d une presse réduisant le volume total de déchet mais n'éliminant pas les effluents chimiques secondaires. Ce dispositif est, en raison des investissements nécessaires, plus particulièrement destiné aux collectivités. En outre, se pose toujours le problème d'éliminer la solution désinfectante. Quant aux dispositifs mettant en oeuvre des solutions physiques tels que des traitements par radiations ionisantes, ils sont très encombrants, onéreux et nécessitent une protection des personnes et de l'environnement.

D'autres dispositifs, conformes par exemple à celui décrit dans le brevet DE-A-3.324.939, comportent une enceinte de décontamination contenant un liquide de nettoyage soumis à intervalles réguliers à un gaz enrichi en ozone. Ces dispositifs incluent généralement en outre un générateur à ultra-sons. Ces procédés mixtes, chimiques, gazeux et ultra-sons sont coûteux et ne donnent pas toujours entière satisfaction. Un procédé similaire est décrit également dans le brevet EP-A-202.366.

Enfin les dispositifs les plus proches de celui décrit ci-après sont ceux par exemple du brevet WO-A-8.605.100 dans lequel on décrit un procédé et un dispositif pour la stérilisation de matériel médical au moyen d ozone à l'état gazeux. Le dispositif décrit est relativement complexe notamment parce que le résultat final désiré est une stérilisation. Ce dispositif comprend une enceinte à l'intérieur de laquelle sont disposés les déchets à stériliser, un générateur d'ozone relié à une source d'oxygène, une pompe à vide, des moyens de chauffage et des moyens de destruction de l'ozone produit. Le procédé de stérilisation s'accompagne donc d'une mise sous vide de l'enceinte et éventuellement d'une humidification de cette dernière. Ce procédé et ce dispositif, malgré leurs avantages à savoir l'absence de pollution, présentent deux inconvénients majeurs. Tout d'abord, l'ozone est produit à partir d'oxygène pur ce qui, de ce fait, entraîne un certain nombre de risques au niveau de l'installation, notamment en raison du fait que l'on traite des déchets susceptibles de contenir de la graisse et pouvant entraîner des explosions lors de la mise en contact avec de l'oxygène. En outre, ce dispositif entraîne un fonctionnement du générateur d'ozone à partir d'un flux gazeux à fort taux d'humidité et de pollution impliquant une baisse de rendement du générateur.

Le brevet FR-A-2.611.343 décrit un dispositif de stérilisation similaire à celui décrit précédemment dans lequel l'ozone est toujours produit à partir d'oxygène et on retrouve un couplage direct entre l'enceinte de stérilisation et l'ozoneur, l'augmentation de la concentration en ozone s'effectuant par le chauffage de l'oxygène ozoné à l'entrée de l'enceinte de stérilisation.

Enfin, on connaît par le brevet FR-A-1.137.268 une installation de base du type comportant un générateur d'air ozoné intégré à l'intérieur de l'enceinte de décontamination et permettant grâce au flux d'air ozoné généré, d'aseptiser des ouvrages. Cependant ce type d'installation ne permet pas d'obtenir des concentrations élevées ou variables en ozone.

Le but de la présente invention est donc de pallier ces inconvénients en proposant un dispositif de traitement, en particulier de décontamination éliminant tout risque d'explosion du fait de l'absence d'une source d'oxygène et donc travaillant non pas à partir d'oxygène ozoné mais à partir d'air ozoné et permettant, grâce à sa configuration simple mais performante, de travailler à des concentrations en ozone variables mais élevées grâce à des moyens de génération rapides et performants sans augmenter le coût de l'installation.

L'invention concerne à cet effet un dispositif pour le traitement, en particulier la décontamination, la désinfection, la désodorisation de matériaux liquides, gazeux ou solides tels que des déchets, notamment ceux provenant du milieu médical, du type comportant une enceinte de traitement ouverte ou fermée contenant le milieu à traiter, un générateur d'ozone susceptible d'alimenter cette enceinte en gaz ozoné, un destructeur d'ozone, susceptible de détruire l'ozone dans le gaz ozoné après et/ou pendant le traitement, caractérise en ce qu'il comprend en outre un accumulateur de gaz ozoné intercalé entre le générateur d'ozone et l'enceinte contenant le milieu à traiter, cet accumulateur étant susceptible d'établir avec le générateur d'ozone, par l'intermédiaire de tubulures appropriées, un circuit fermé de manière à obtenir rapidement des concentrations élevées et variables en ozone dans le gaz destiné à circuler dans l'enceinte contenant le milieu à traiter.

Dans le cas d'une décontamination, on peut, grâce à cette configuration, faire varier la concentration en ozone dans le gaz ozoné circulant à l'intérieur de l'enceinte de traitement en fonction du degré de contamination des déchets à décontaminer, de la durée du cycle de décontamination et de l'instant auquel on injecte cet air ozoné au cours du cycle de décontamination. On peut ainsi obtenir un dispositif dont le fonctionnement est modulable, ce qui assure une décontamination parfaite avec une bonne maîtrise des dépenses d'énergie.

D'autres caractéristiques et avantages de l'invention apparaîtront encore à la lecture de la description qui suit en référence aux dessins joints, lesquels description et dessins sont donnés surtout à titre d'exemples. Dans ces dessins :
la figure 1 représente une vue schématique d'un dispositif conforme à l'invention,
la figure 2 représente une vue en perspective d'un mode de réalisation de moyens d'introduction des déchets dans l'enceinte de décontamination, et
la figure 3 représente une vue en coupe selon AA de la figure 2.

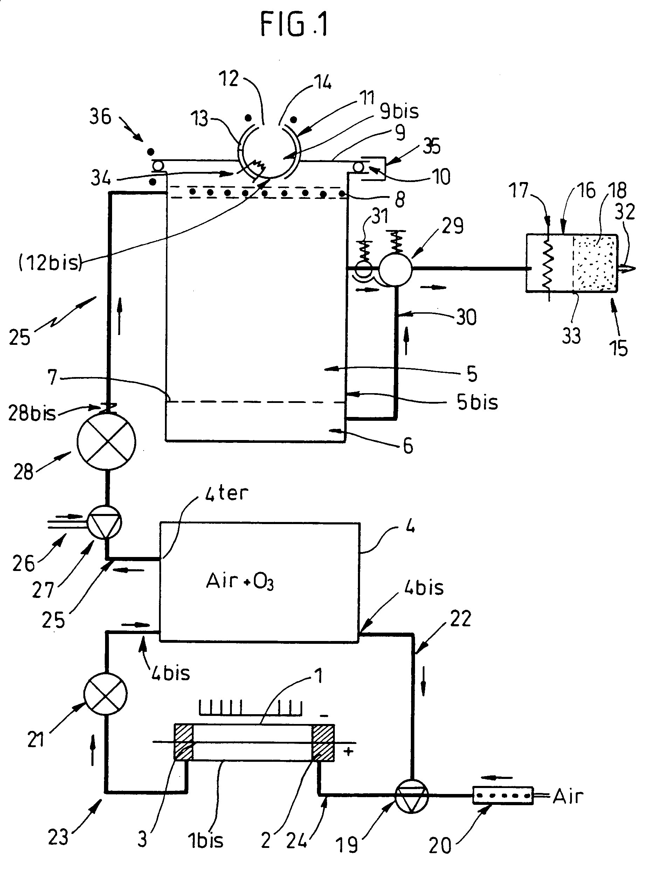

Conformément à l'invention telle que représentée aux figures 1 à 3, le dispositif de décontamination comprend un générateur d ozone 1, un accumulateur de gaz ozoné 4, une enceinte étanche de décontamination des déchets 5 par action du gaz ozoné produit par le générateur 1 et un destructeur d'ozone 16. Ces constituants principaux sont reliés les uns aux autres par un ensemble de tubulures, de vannes et de pompes permettant de mettre en oeuvre le procédé de décontamination.

Le générateur d'ozone 1 consiste en un système tubulaire du type condensateur cylindrique comprenant une électrode intérieure constituée par un fil 3 de faible diamètre entourée coaxialement d'une électrode extérieure 1bis. Ce système fil cylindre présente l'avantage d'être de dimension réduite. Le fil constituant l'électrode intérieure est maintenu à une haute tension positive ou négative, le cylindre extérieur 1a étant relié quant à lui à la masse. Le gaz entre par la canalisation 24 et sort par la tubulure 23 et circule entre les deux électrodes axialement. Ce système fil cylindre constitue le corps de l'ozoneur, le centrage du fil étant assuré par deux embouts 2 isolants percés en leur centre pour permettre l'écoulement du gaz et le centrage de l'électrode intérieure. Ces matériaux isolants obstruant l'extrémité de l'électrode extérieure laissent entrer ou sortir le flux gazeux par un orifice relié aux tubulures de circulation d'air. Ces embouts isolants sont eux-mêmes insérés dans deux pièces terminales dont l'une au moins est mobile et/ou équipée d'un contact métallique relié à la haute tension. De ce fait, on obtient un générateur d'ozone de faible encombrement qui peut être démonté aisément pour être remplacé ou nettoyé. On notera que, dans le cas représenté à la figure 1, le gaz qui parcourt le générateur est de l'air qui a en plus été desséché au moyen d'un dessiccateur 20. Le fait de dessécher l'air permet d augmenter l'efficacité du générateur d ozone 1. Ainsi constitué, le générateur 1 représente un volume cylindrique fermé pouvant être traversé suivant son grand axe par un flux de gaz lorsqu'on applique entre son axe et la surface de sa paroi de révolution un champ électrique continu. De ce fait, les décharges couronne générées provoquent la formation d ozone dans le flux gazeux à partir de l'oxygène de l'air qui le parcourt. De façon à augmenter le rendement du tube générateur en évitant son échauffement, un dispositif de refroidissement (non représenté) peut éventuellement lui être annexé.

Ce générateur d'ozone 1 est relié au moyen d'une tubulure 23 à l'entrée de l'accumulateur de gaz ozone 4 et par une tubulure 22 à la sortie de l'accumulateur de gaz ozone 4. De ce fait, il est possible d'établir un circuit fermé entre l accumulateur de gaz ozone 4 et le générateur d'ozone 1. Cet accumulateur de gaz ozone 4 se présente sous forme d'un réservoir étanche 4 en acier inoxydable ou tout autre matériau approprié. Son volume est établi de façon correcte en fonction des caractéristiques du générateur d'ozone 1 d'une part et du volume de l'enceinte de décontamination 5 d'autre part. La tubulure 23 reliant la sortie du générateur d'ozone 1 à l'entrée 4bis de l'accumulateur de gaz ozoné 4 comporte une pompe 21 qui facilite l'amenée du gaz ozoné du générateur 1 à l'accumulateur 4 et permet le pompage de l'air ambiant pour l amener au générateur 1. La deuxième tubulure qui relie l'accumulateur de gaz ozone 4 à l'entrée du générateur d'ozone 1 comporte une vanne trois voies 19. Cette vanne, disposée à l'intersection de la tubulure 22 reliant l'accumulateur de gaz ozoné 4 au générateur d'ozone 1 et de la tubulure 24 reliant la source de gaz à l'entrée du générateur d'ozone 1, permet d'admettre dans l'ensemble générateur 1 soit un gaz provenant de l'accumulateur de gaz ozoné 4 soit un gaz provenant d'une source extérieure aspirée au moyen de la pompe 21 après passage à travers un dessiccateur 20.

Ceci ne constitue qu'un mode de réalisation de l'invention. En effet, en raison de la configuration très simple du générateur d'ozone 1, on peut imaginer un dispositif dans lequel on dispose plusieurs générateurs d'ozone 1 en parallèle, certains étant alimentés par le gaz ozoné provenant de l'accumulateur de gaz ozoné 4, d'autres étant approvisionnés directement par la source de gaz externe. De ce fait, on obtient très rapidement des concentrations élevées en ozone dans le gaz ozoné stocké à l'intérieur de l'accumulateur de gaz ozoné 4. En outre, ce type d'installation permet de travailler directement avec l'air ambiant en raison d'un rendement extrêmement élevé du générateur d'ozone 1 du fait de la configuration de l'ensemble. De ce fait, on supprime un inconvénient majeur des dispositifs existants qui s approvisionnent tous à partir d'oxygène pur, ce qui nécessite une source d'oxygène appropriée à proximité. Dans l'installation décrite ici, le dispositif est entièrement autonome et ne nécessite pas de source de gaz particulière. On notera que l'accumulateur de gaz ozoné 4 comprend un troisième orifice 4ter qui lui permet, au moyen d'une tubulure 23, d'être relié à l'enceinte de décontamination de déchets 5 proprement dite. Cette liaison s'effectue par l'intermédiaire de la tubulure 25, de la vanne trois voies 27 et de la pompe 28.

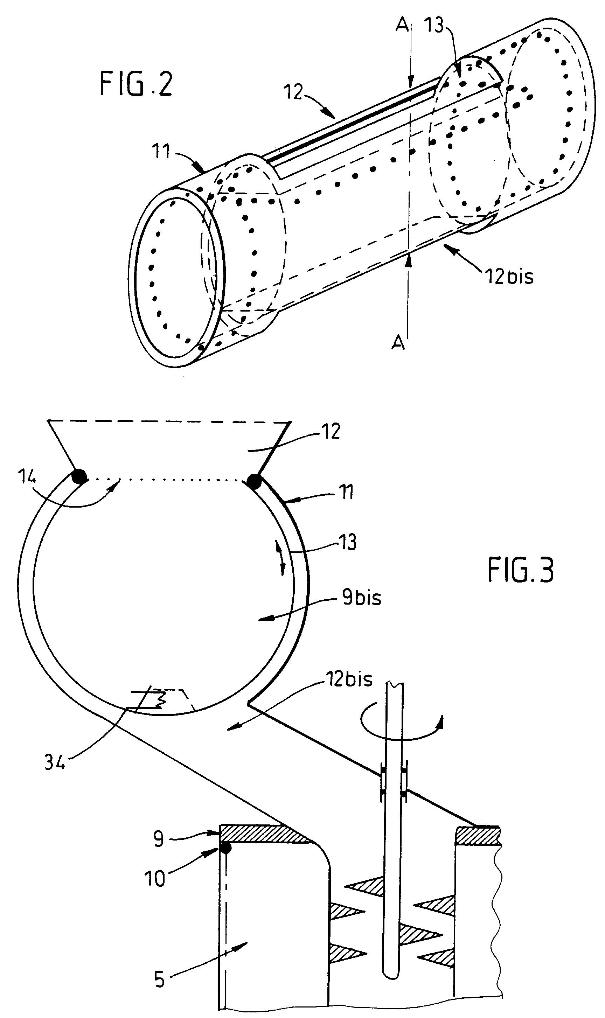

L'enceinte de décontamination des déchets 5 est constituée d'un corps 5bis en acier inoxydable ou en tout autre matériau approprié et comporte à sa partie inférieure une grille 7 formant double fond et délimitant un espace inférieur 6 destiné à la récupération des gaz de traitement. Les déchets à traiter sont placés au-dessus de cette grille et l'enceinte 5 est fermée par un couvercle 9 verrouillé au moyen d'une serrure de préférence à gâche électrique 35, l'étanchéité étant assurée par un joint torique 10. Ce couvercle 9 peut comporter un sas d'entrée 9bis permettant l'introduction en continu ou quasi-continu de déchets à l'intérieur de l'enceinte de décontamination 5, c'est-à-dire y compris lors d un cycle de décontamination. Ce sas d'entrée 9bis peut par exemple être réalisé conformément à la figure 2.

Dans ce cas, ce sas d'entrée affecte la forme d'un tiroir rotatif cylindrique constitué d'une double enveloppe de manière à délimiter un cylindre externe 11 solidaire du couvercle et comportant des orifices supérieurs 12 et inférieurs 12bis par lesquels les déchets pourront être introduits et évacués d'un cylindre interne 13 rotatif comportant un seul orifice 14 sur sa surface de révolution. Grâce à ce mode de réalisation du sas, la concordance de l'orifice 12 avec l'orifice 14 permet l'introduction des déchets dans le cylindre interne 13 sans relargage à l'extérieur de l'air fortement enrichi en ozone de l'ensemble 5 où sont contenus les déchets en cours de traitement. Ensuite, la rotation à 180° du cylindre interne 13 faisant concorder l'orifice 14 du cylindre interne 13 et l'orifice inférieur 12bis du cylindre externe 11, permet aux déchets déposés dans le cylindre interne 13 de tomber dans l'enceinte de traitement 5 par simple gravité sans que le gaz de traitement puisse s'échapper à l'extérieur. En outre, la concordance des orifices 12bis et 14 établit dans le corps du cylindre rotatif interne 13 une atmosphère désinfectante comparable à celle de l'enceinte 5 assurant son auto-décontamination après passage des déchets polluants ayant contaminé sa surface. De plus, ce tiroir et notamment le cylindre interne 13 peut être muni de moyens de chauffage 34 qui permettent la destruction d'ozone à l'intérieur de ce cylindre interne 13 avant que celui-ci soit mis en communication avec l'extérieur pour le remplissage des déchets. Les déchets introduits dans le cylindre interne 13 parviennent dans l'enceinte de décontamination 5 après avoir eventuellement été broyés au moyen d'un dispositif de broyage disposé dans l'enceinte de décontamination 5. Ce dispositif de broyage est de préférence constitué comme suit :

Compte tenu de la nature hétérogène des déchets (aiguilles, poches de sang, tubulures diverses, caoutchouc, compresses, etc...), un dispositif de broyage peut être annexé à la sortie du sas dans l'enceinte de traitement.

Ce dispositif peut être avantageusement réalisé par l'utilisation d'un arbre tournant à très haute vitesse angulaire et portant un ou plusieurs couteaux réalisés en matière appropriée et à constituer autour de cet ensemble tournant un carter hémisphérique portant à sa surface un certain nombre de couteaux fixes ou d'aspérités ainsi que des orifices calibres.

L'ensemble ainsi constitué permet de dilacérer les textiles et de casser les constituants durs des déchets et de les éjecter dans le volume de traitement au niveau des injecteurs d'air ozoné augmentant la surface de contact gaz-déchets, améliorant ainsi l'efficacité et la rapidité du traitement tout en donnant aux résidus traités un aspect de déchets ordinaires.

Bien évidemment tout autre mode de réalisation de moyens d'introduction des déchets dans l'enceinte de décontamination peut être envisagé. Il est également possible de prévoir à l'intérieur de l'enceinte un récipient supplémentaire amovible, interchangeable et à usage unique. Ce récipient reçoit les déchets et présente une forme conique (deux cônes renverses) avec un étranglement sensiblement dans sa partie médiane. En outre, il est susceptible d'être ferme à ses deux extrémités au moyen de couvercles venant fermer le récipient par simple emboutissage. De ce fait, on peut imaginer une procédure de décontamination dans laquelle les déchets sont stockés à l'intérieur de ce container ou récipient, décontaminés par envoi de gaz ozoné à l'intérieur dudit container. Une fois la décontamination terminée, le récipient est fermé de manière définitive et l'ensemble est extrait de l'enceinte de décontamination 5 après destruction de l'ozone à l'intérieur de ladite enceinte et le container est directement évacué vers une décharge. On élimine par ce moyen tout risque de blessure d'un utilisateur qui aurait à manipuler ces déchets.

Le dispositif représenté à la figure 1 comporte en outre un destructeur d'ozone. Ce destructeur est constitué d'un corps 16 généralement cylindrique fermé à ses deux extrémités et relié d'un côté à la tubulure de sortie 31 reliant ce corps 16 à l'enceinte de décontamination 5 par l'intermédiaire d'une vanne anti-surpression automatique 29 et de l'autre côté à la tubulure d'évacuation générale 32 qui mène à l'atmosphère. Ce corps est séparé en deux chambres au moyen d'une grille 33. La première chambre comporte une résistance 17 chauffée dès que le dispositif est mis sous tension. Le rôle de cette résistance est de détruire l'ozone avant toute évacuation de gaz vers l'extérieur. La deuxième chambre 15 comporte quant à elle un filtre à sable 18 destiné d'une part à parachever la destruction de l'ozone qui aurait pu échapper à l'action de la résistance chauffée 17 et d'autre part à empêcher la sortie de particules polluantes lors de la purge de l'enceinte 5. Bien évidemment, la description ci-dessus n'est qu'un exemple de réalisation d'un destructeur d'ozone bien connu dans l'état de la technique. On notera qu'une tubulure 30 disposée entre la vanne anti-surpression automatique 29 et l'enceinte de décontamination 5 permet de ramener une partie du gaz ozoné vers l'enceinte de décontamination 5 lorsqu'une surpression ponctuelle s'est produite mais que le cycle de destruction de gaz n'a pas encore commencé.

En conséquence, avec un dispositif du type décrit à la figure 1, le mode de fonctionnement du dispositif et le procédé de décontamination sont les suivants : le générateur d ozone 1 est placé sous tension et est relié à l'atmosphère de manière à établir un circuit de production d'air fortement enrichi en ozone à partir de l'oxygène contenu dans l'air atmosphérique. Bien évidemment, on peut imaginer toute autre source gazeuse, l'air restant le gaz le plus pratique à utiliser. Cet enrichissement progressif en ozone de l'air ambiant est obtenu en établissant une boucle fermée entre le générateur d'ozone 1 et l'accumulateur de gaz ozoné 4. Ainsi, l'air pompé au moyen de la pompe 21 et desséché par le dessiccateur 20 est, par l'intermédiaire de la vanne trois voies 19, acheminé au moyen de la tubulure 24 à l'intérieur du générateur d'ozone 1 qu'il traverse pour ensuite passer dans la tubulure 23 et aboutir par l'intermédiaire de l'orifice 4bis à l'intérieur de l'accumulateur de gaz ozoné 4. Ce gaz ozoné une fois introduit à l'intérieur de l'accumulateur de gaz ozoné 4 peut avoir deux possibilités.

Soit, dans le cas où on veut augmenter encore la concentration de ce gaz ozoné, on retourne, par l'intermédiaire de l'orifice 4bis et de la tubulure 22 puis passage par la vanne trois voies 19, vers le générateur d'ozone 1 et on réitère cette boucle jusqu'à ce que la concentration en ozone du gaz soit suffisante. Soit on introduit de nouveau de l'air en orientant la vanne trois voies 19 sur la tubulure placée à la pression atmosphérique. Lorsqu'une concentration suffisante est atteinte, c'est-à-dire lorsque les passages successifs de gaz ozoné contenus dans l'accumulateur de gaz ozoné 4 au travers du tube générateur d'ozone 1 ont permis d'obtenir une concentration en ozone de l'ordre de 10 000 PPM, le gaz ozoné est alors pompé au moyen de la pompe 28 disposée sur la tubulure 25 pour être acheminé en direction de l'enceinte de décontamination 5.

Le mélange de gaz ozoné contenu dans l'accumulateur de gaz ozoné 4 est alors injecté dans l'enceinte de décontamination 5. Cette injection s'effectue au moyen d'un diffuseur périphérique 8 placé dans la partie haute de l'enceinte de décontamination 5. Des injections successives de ce gaz ozoné entraînent une légère compression du gaz et on aboutit généralement à une pression dans l'enceinte de décontamination 5 de l'ordre de 2 à 3 bars. Des moyens de purge de l'enceinte de décontamination 5 sont également prévus. Ils peuvent être notamment constitués par une tubulure d'admission 26 reliée à l'air atmosphérique permettant l'admission d'air au moyen de la pompe 28 dans l'enceinte de décontamination 5 puis vers le piège à ozone ou destructeur d'ozone au travers de la vanne trois voies 29.

Lorsque l'enceinte de décontamination 5 présente une concentration en gaz ozoné, en particulier en ozone, suffisante, les cycles de décontamination commencent de manière à rendre maximale la mise en contact des déchets avec l'air ozoné. Ces déchets peuvent être déjà placés dans l'enceinte ou au contraire introduit au moyen du sas d'alimentation. Généralement, il est prévu qu'à chaque introduction de déchets dans l'enceinte 5, par l'intermédiaire du sas d'entrée 9bis, la pompe 28 prélève une quantité d'air ozoné à forte concentration d'ozone, air ozone qui va balayer la surface du nouveau dépôt de déchets. La répétition de cette procédure engendre une augmentation de la pression locale 5 en fonction des performances de la pompe 28 et de son clapet anti-retour 28bis. Cette pression est limitée par ailleurs par le tarage de la valve de sécurité 29. L'ozone injecté à chaque introduction de déchets a tendance, du fait de sa densité, a s accumuler au fond de l'enceinte 5. Pour assurer l'efficacité du traitement des dernières couches déposées qui ont été soumises à un moins grand nombre d'injection d'ozone, les automatismes annexes imposent la réalisation d'un nombre suffisant de cycles d'injection hors de toute nouvelle manoeuvre du sas et, donc, d'introduction de déchets puisque celui-ci est verrouillé.

Lors de ce programme de traitement en absence de toute introduction supplémentaire de déchets, ces déchets, en particulier s'ils ont été déchiquetés ou broyés, peuvent être remués par un dispositif d'agitation entraînant le maximum de contact entre leur surface et l'air ozoné. Durant cette deuxième phase de traitement, le sas est verrouillé en position fermée et l'impossibilité d'ouverture du couvercle 9 est maintenue, comme depuis la mise en marche de l'appareil, par une serrure automatique 35. Le déverrouillage de la fermeture 35 de par la programmation de l'appareil ne peut être obtenu qu'après réalisation complète de l'ensemble des cycles des deux programmes successifs, assurant ainsi la sécurité de décontamination. L'automatisme de pilotage de ces cycles assure également le verrouillage de la serrure 35 si l'ensemble des phases des deux programmes prévus ne s'est pas déroulé complètement une alarme éventuellement sonore ou visuelle étant déclenchée.

Enfin, lorsque les cycles de décontamination sont terminés, on entame le cycle de destruction du gaz ozoné. Pour pratiquer cette élimination de l'ozone contenu dans le système, on purge à partir de l'air ambiant insufflé dans l'enceinte de décontamination 5 au moyen de la pompe 28 et de la vanne 27 reliée par l'intermédiaire de la tubulure 26 à l'air ambiant. De ce fait, on chasse l'air chargé en ozone par l'intermédiaire de la tubulure 31 et passage à travers la vanne 29 dans le destructeur d'ozone 16 décrit ci-dessus. Là, le gaz ozoné est chauffé et filtré avant d'être rejeté dans l'atmosphère. Pour parfaire la sécurité du dispositif, il est prévu un certain nombre d'automatismes annexés aux dispositifs qui font que, dès la mise en route de l'appareil, la résistance chauffante 17 du destructeur d'ozone 16 est allumée et que, si celle-ci présente un dysfonctionnement, la pompe basse pression 21 et le fil 3 du tube générateur d'ozone 1 sont mis hors tension. La pompe haute pression 28 entraîne une purge de l'ensemble du système par de l'air provenant de la tubulure d'admission 26 à travers la vanne trois voies 27.

En outre, des indicateurs colorés très sensibles sont disposés à chaque endroit où l'ozone pourrait s'échapper, avertissant l'utilisateur de la nécessité de pratiquer une ventilation énergique du local au cas où une sortie d'ozone accidentelle se serait produite malgré les dispositifs de sécurité actif et passif.

Dans l'exemple de réalisation décrit ci-dessus, l'enceinte de décontamination est une enceinte fermée étanche. En fonction des applications, cette enceinte peut également être une enceinte ouverte. Cette enceinte peut contenir un milieu à traiter se présentant sous forme de matériaux solides ou liquides ou gazeux. Ainsi, dans le cas de traitement des eaux, le milieu à traiter sera contenu soit dans des canalisations, soit dans des conteneurs généralement ouverts. Dans le cas de déchets, par exemple ménagés, stockés en vrac dans des conteneurs, l'enceinte sera généralement ouverte sur sa face du dessus. Le terme enceinte doit donc être compris comme un terme très général pouvant désigné soit une enceinte fermée, soit une enceinte ouverte, cette enceinte pouvant contenir des matériaux liquides, solides ou gazeux, ces matériaux devant être traités par un traitement de surface qui peut être une décontamination ou tout autre traitement susceptible d'être obtenu au moyen de l'utilisation d'un gaz ozoné. Par autre traitement, on entend notamment la désinfection ou la désodorisation du milieu à traiter En conséquence de ce qui vient d'être dit, le dispositif décrit ci-dessus ne constitue qu'un mode de réalisation de l'invention cité à titre d'exemple. L'intérêt majeur de ce type d'installation est la possibilité de pouvoir faire varier à volonté la concentration en ozone du gaz injecté à l'intérieur de l'enceinte de traitement 5. De ce fait, on peut moduler à souhait les cycles de décontamination en fonction de l'origine des déchets. En outre, ce dispositif peut fonctionner quasiment en continu dans la mesure où la production de gaz ozoné et le traitement sont constitués par des circuits totalement indépendants et que l'alimentation en déchets peut se faire en plusieurs étapes. En conséquence, la production de gaz ozoné peut se produire même lorsqu'on décontamine des déchets. Enfin, par le biais de générateurs d'ozone disposés en parallèle, on peut aboutir à un dispositif peu onéreux mais particulièrement performant permettant le mise en oeuvre de cycles de décontamination particulièrement courts.

## Revendications

1. Dispositif pour le traitement, en particulier la décontamination, la désinfection, la désodorisation de matériaux liquides, gazeux ou solides, tels que des déchets, en particulier ceux provenant du milieu médical, du type comportant une enceinte (5) de traitement du milieu à traiter, un générateur d'ozone (1) susceptible d'alimenter cette enceinte (5) en gaz ozoné, un destructeur d'ozone (16) susceptible de détruire l'ozone du gaz ozoné contenu dans l'enceinte de traitement (5),
caractérisé en ce qu'il comprend en outre un accumulateur de gaz ozoné (4) intercalé entre le générateur d'ozone (1) et l'enceinte de traitement (5), cet accumulateur (4) étant susceptible d'établir avec le générateur d'ozone (1) par l'intermédiaire de tubulures (22, 23, 24) appropriées, un circuit fermé de manière à augmenter et faire varier rapidement la concentration en ozone dans le gaz destiné à circuler dans l'enceinte de décontamination (5).

2. Dispositif selon la revendication 1,
caractérisé en ce que l'accumulateur de gaz ozoné (4) comprend en outre une liaison directe (25) avec l'enceinte de traitement, cette liaison étant susceptible d'être obturée.

3. Dispositif selon la revendication 1,
caractérisé en ce que le générateur d'ozone (1) est du type fil-cylindre et présente un corps de générateur monté amovible sur un support comportant des moyens de raccordement à une source de fluide gazeux et à une source de haute tension.

4. Dispositif selon la revendication 1,
caractérisé en ce que le générateur d'ozone (1) est approvisionné en gaz soit par une source de gaz externe, soit par le gaz ozoné provenant de l'accumulateur (4), le passage d'une source à une autre s effectuant par basculement automatique d'un organe d'obturation (19) disposé entre l accumulateur de gaz ozoné (4) et le générateur d'ozone (1).

5. Dispositif selon l'une des revendications 1 à 4,
caractérisé en ce que le générateur d'ozone est constitué d'au moins deux générateurs d'ozone disposés en parallèle, l'un étant approvisionné par le gaz ozone provenant de l accumulateur d'ozone (4), l'autre étant approvisionné par une source de gaz externe ou par le gaz ozoné provenant de l accumulateur d'ozone (4).

6. Dispositif selon l'une des revendications 1 à 5,
caractérisé en ce que l'enceinte de traitement (5) comporte des moyens d'alimentation des déchets (9bis) permettant l'introduction des déchets dans l'enceinte de décontamination (5) y compris lors d'un cycle de fonctionnement du dispositif de traitement, en particulier de décontamination.

7. Dispositif selon la revendication 6,
caractérisé en ce que lesdits moyens d'alimentation en déchets (9bis) de l'enceinte (5) sont constitués par un réceptacle disposé de préférence au sommet de l'enceinte et monté solidaire du couvercle (9) fermant ladite enceinte (5), ledit réceptacle étant constitué d'une double enveloppe (11, 13) délimitant une cavité, l'enveloppe interne (13) étant susceptible d'être entraînée en rotation à l'intérieur de l'enveloppe externe (11) et comportant une ouverture (14) susceptible lors de sa rotation de venir en regard soit d'une ouverture supérieure (12) de l'enveloppe externe (18) pour permettre l'introduction des déchets dans ledit réceptacle, soit en regard d une ouverture inférieure (12bis) de l'enveloppe externe (11) pour permettre le passage des déchets du réceptacle à l'enceinte de décontamination (5).

8. Dispositif selon la revendication 7,
caractérisé en ce que l'enveloppe interne (13) comporte des moyens de destruction de l'ozone tels que des moyens de chauffage (34).

9. Dispositif selon la revendication 7,
caractérisé en ce que l'enceinte de traitement (5) comporte en outre des moyens de broyage des déchets disposés soit dans le réceptacle d'introduction des déchets à l'intérieur de l'enceinte de décontamination, soit dans l'enceinte de décontamination (5) proprement dite.

10. Dispositif selon l'une des revendications 1 à 9,
caractérisé en ce que le destructeur d'ozone est constitué par un corps fermé à ses deux extrémités et disposé à l'extérieur de l'enceinte de traitement (5) et relié à cette dernière, ledit corps étant divisé en deux chambres, l'une contenant des moyens de destruction de l'ozone tels que des moyens de chauffage (17), l'autre contenant des moyens de retenue de l'ozone tels que des moyens de filtration (18).

11. Dispositif selon l'une des revendications 1 à 10,
caractérisé en ce qu'un réceptacle jetable à usage unique susceptible d'être obturé à ses deux extrémités après décontamination est disposé dans l'enceinte de décontamination (5) avant chaque cycle de décontamination et reçoit les déchets.

12. Procédé de traitement, en particulier de décontamination des matériaux de préférence solides tels que des déchets en particulier provenant du milieu médical disposés dans une enceinte de traitement soumis à l'action d'un gaz ozoné produit par un générateur d'ozone (1) et éliminé par un destructeur d'ozone (16),
caractérisé en ce qu'on intègre entre l'enceinte de traitement (5) et le générateur d'ozone (1) un accumulateur de gaz ozoné (4) susceptible d'établir avec le générateur d'ozone (1) un circuit fermé de manière à enrichir le gaz ozoné en ozone.

## Claims

1. Device for the treatment, particularly the decontamination, the disinfection, the deodorization of liquid, gaseous, or solid materials such as waste, particularly that coming from the medical environment, of the type comprising an enclosure (5) with ozonized gas, an ozone destroyer (16) capable of destroying the ozone of the ozonized gas contained in the treatment enclosure (5), characterized in that it further includes an accumulator of ozonized gas (4) inserted between the ozone generator (1) and the treatment enclosure (5), this accumulator (4) being capable of establishing with the ozone generator (1), via suitable pipes (22, 23, 24), a closed circuit so as rapidly to increase and vary the ozone concentration in the gas intended to circulate in the decontamination enclosure (5).

2. Device according to claim 1, characterized in that the accumulator of ozonized gas (4) further comprises a direct connection (25) with the treatment enclosure, this connection being capable of being blocked.

3. Device according to claim 1, characterized in that the ozone generator (1) is of the wire-cylinder type and has a generator body mounted removably on a support comprising means for connection to a gaseous-fluid source and to a high-voltage source.

4. Device according to claim 1, characterized in that the ozone generator (1) is supplied with gas either by an external gas source or by the ozonized gas coming from the accumulator (4), the passage from one source to another being effected by automatic tipping of a blocking agent (19) disposed between the accumulator of ozonized gas (4) and the ozone generator (1).

5. Device according to one of the claims 1 to 4, characterized in that the ozone generator is made up of at least two ozone generators disposed in parallel, one being supplied by the ozonized gas coming from the ozone accumulator (4), the other being supplied by an external gas source or by the ozonized gas coming from the ozone accumulator (4).

6. Device according to one of the claims 1 to 5, characterized in that the treatment enclosure (5) comprises means for feeding the waste (9bis) permitting the introduction of the waste into the decontamination enclosure (5), including at the time of an operating cycle of the treatment, particularly decontamination, device.

7. Device according to claim 6, characterized in that said waste feeding means (9bis) of the enclosure (5) are made up of a receptacle preferably disposed at the top of the enclosure and mounted integral with the cover (9) closing said enclosure (5), said receptacle being made up of a double envelope (11, 13) delimiting a cavity, the inner envelope (13) being capable of being driven rotatingly within the outer envelope (11) and comprising an aperture (14) capable at the time of its rotation of coming opposite either an upper aperture (12) of the outer envelope (18) [sic.] for permitting the introduction of waste into said receptacle, or opposite a lower aperture (12bis) of the outer envelope (11) for permitting the passage of the waste from the receptacle to the decontamination enclosure (5).

8. Device according to claim 7, characterized in that the inner envelope (13) comprises means for destruction of the ozone, such as heating means (34).

9. Device according to claim 7, characterized in that the treatment enclosure (5) further comprises means for grinding the waste disposed either in the receptacle for introduction of the waste into the interior of the decontamination enclosure or in the decontamination enclosure (5) proper.

10. Device according to one of the claims 1 to 9, characterized in that the ozone destroyer is made up of a body closed at both ends and disposed outside the treatment enclosure (5) and connected to the latter, said body being divided into two chambers, one containing means for destruction of the ozone such as heating means (17), the other containing means for retaining the ozone such as filtration means (18).

11. Device according to one of the claims 1 to 10, characterized in that a disposable receptacle for one-time use, capable of being blocked at both ends after decontamination, is disposed in the decontamination enclosure (5) before each decontamination cycle and receives the waste.

12. Process for treatment, particularly for decontamination of preferably solid materials such as waste particularly coming from the medical environment disposed in a treatment enclosure subjected to the action of an ozonized gas produced by an ozone generator (1) and eliminated by an ozone destroyer (16), characterized in that an accumulator of ozonized gas (4) capable of establishing a closed circuit with an ozone generator (1) so as to enrich the ozonized gas with ozone is integrated between the treatment enclosure (5) and the ozone generator (1).

## Patentansprüche

1. Vorrichtung für die Behandlung, insbesondere die Dekontaminierung, Desinfektion, Desodorierung von flüssigen, gasförmigen oder festen Materialen, wie Abfällen, insbesondere solche, die aus dem medizinischen Bereich stammen, des Typs enthaltend ein Gefäss (5) zur Behandlung des Behandlungsmediums, einen Ozongenerator (1), welcher dieses Gefäss (5) mit einem ozonierten Gas speisen kann, einen Ozonzerstörer (16), welcher fähig ist, das Ozon des ozonierten Gases zu zerstören, welches sich im Behandlungsgefäss (5) befindet, dadurch gekennzeichnet, dass sie im weiteren einen Akkumulator für ozoniertes Gas (4) enthält, welcher zwischen dem Ozongenerator (1) und dem Behandlungsgefäss (5) eingefügt ist, wobei dieser Akkumulator (4) fähig ist, mittels zweckmässigen Rohren (22, 23, 24) einen geschlossenen Kreislauf zu bilden, derart, dass die Ozonkonzentration im Gas, welches zum Zirkulieren im Dekontaminationsgefäss (5) bestimmt ist, erhöht und schnell verändert wird.

2. Vorrichtung gemäss Anspruch 1, dadurch gekennzeichnet, dass der Akkumulator (4) für ozoniertes Gas im weiteren eine direkte Verbindung (25) mit dem Behandlungsgefäss aufweist, wobei diese Verbindung verschlossen werden kann.

3. Vorrichtung gemäss Anspruch 1, dadurch gekennzeichnet, dass der Ozongenerator (1) vom Draht-Zylinder-Typ ist und einen Generatorkörper aufweist, der beweglich auf einer Stütze montiert ist, welche Mittel zur Verbindung mit einer Flüssiggasquelle und einer Hochspannungsquelle trägt.

4. Vorrichtung gemäss Anspruch 1, dadurch gekennzeichnet, dass der Ozongenerator (1) mit Gas versorgt wird, entweder durch eine externe Gasquelle oder durch ein ozoniertes Gas, welches aus dem Akkumulator (4) stammt, wobei der Durchgang von einer Quelle zur andern durch eine automatische Schaltung mittels eines Verschlussorganes (19) verwirklicht wird, welches zwischen dem Akkumulator (4) für ozoniertes Gas und dem Ozongenerator (1) angeordnet ist.

5. Vorrichtung gemäss einem der Ansprüche 1 bis 4, dadurch gekennzeichnet, dass der Ozongenerator aus mindestens zwei Ozongeneratoren besteht, welche parallel angeordnet sind, wobei der eine mit ozoniertem Gas versorgt wird, welches aus dem Ozonakkumulator (4) stammt und der andere durch eine externe Gasquelle oder durch ozoniertes Gas, das aus dem Ozonakkumulator (4) stammt, versorgt wird.

6. Vorrichtung gemäss einem der Ansprüche 1 bis 5, dadurch gekennzeichnet, dass das Behandlungsgefäss (5) Mittel zur Zuführung von Abfällen (9bis) trägt, welche die Einführung von Abfällen in das Dekontaminationsgefäss (5) einschliesslich in den Funktionszyklus der Behandlungsvorrichtung, insbesondere für die Dekontamination erlauben.

7. Vorrichtung gemäss Anspruch 6, dadurch gekennzeichnet, dass die genannten Mittel zur Speisung von Abfällen (9bis) des Gefässes (5) aus einem Sammelbecken, welches vorzugsweise oben am Gefäss angeordnet ist und mit dem Deckel (9) verbunden ist, welcher das genannte Gefäss (5) schliesst, bestehen, wobei das genannte Sammelbecken aus einem doppelten Gehäuse (11, 13) besteht, welches einen Hohlraum abgrenzt, wobei das innere Gehäuse (13) dazu bestimmt ist, dass es im Innern des äusseren Gehäuses (11) rotiert und eine Oeffnung (14) trägt, welche dazu bestimmt ist, entweder mit einer oberen Oeffnung (12) des äusseren Gehäuses (18) in Verbindung zu treten, um eine Einführung von Abfällen in den genannten Sammelbehälter zu erlauben, oder um in Verbindung mit einer unteren Oeffnung (12bis) des äusseren Gehäuses (11) zu treten, um den Durchgang der Abfälle des Sammelbehälters in den Dekontaminationsbehälter (5) zu erlauben.

8. Vorrichtung gemäss Anspruch 7, dadurch gekennzeichnet, dass das innere Gehäuse (13) Ozonzerstörungsmittel, wie Heizmittel (34) aufweist.

9. Vorrichtung gemäss Anspruch 7, dadurch gekennzeichnet, dass das Behandlungsgefäss (5) unter anderem ein Mittel zur Zerkleinerung der vorgelegten Abfälle aufweist, entweder im Sammelbehälter zur Einführung der Abfälle in das Innere des Dekontaminationsgefässes oder im Dekontaminationsgefäss (5) selbst.

10. Vorrichtung gemäss einem der Ansprüche 1 bis 9, dadurch gekennzeichnet, dass der Ozonzerstörer aus einem Körper besteht, welcher an seinen beiden Enden geschlossen ist und im Innern des Behandlungsgefässes (5) angeordnet ist und mit dem letzteren verbunden ist, wobei der genannte Körper in zwei Kammern aufgeteilt ist, wobei die eine Ozonzerstörungsmittel wie Heizmittel (7) enthält und die andere Ozonrückhaltemittel, wie Filtermittel (18) enthält.

11. Vorrichtung gemäss einem der Ansprüche 1 bis 10, dadurch gekennzeichnet, dass ein wegwerfbares Sammelgefäss zum einmaligen Gebrauch, das auf seinen beiden Enden nach der Dekontamination geschlossen werden kann, im Dekontaminationsgefäss (5) vor jedem Dekontaminationszyklus und jeder Aufnahme von Abfällen angeordnet wird.

12. Verfahren zur Behandlung, insbesondere zur Dekontamination von Materialien, vorzugsweise Feststoffe wie Abfälle, die aus dem medizinischen Bereich stammen, welche in einem Behandlungsgefäss vorgelegt werden und der Wirkung eines ozonierten Gases ausgesetzt werden, welches durch einen Ozongenerator (1) gebildet und durch einen Ozonzerstörer (16) eliminiert wird, dadurch gekennzeichnet, dass man zwischen dem Behandlungsgefäss (5) und dem Ozongenerator (1) einen Akkumulator für ozoniertes Gas (4) anordnet, welcher dazu bestimmt ist, mit dem Ozongenerator (1) einen geschlossenen Kreislauf zu bilden, derart, dass das ozonierte Gas mit Ozon angereichert wird.
